# EUROPEAN PATENT APPLICATION

(11) **EP 4 009 333 A1**
(43) Date of publication of application: **08.06.2022**
(21) Application number: 20210892.4
(22) Date of filing: 01.12.2020
(51) Int. Cl.: G16H 20/70, A61B 5/377

(54) **METHOD AND SYSTEM FOR PERSONALIZED ATTENTION BIAS MODIFICATION TREATMENT BY MEANS OF NEUROFEEDBACK MONITORING**

(71) Applicant: Koninklijke Philips N.V., 5656 AG Eindhoven (NL)
(72) Inventor: VAN DOOREN, Marieke, 5656 AE Eindhoven (NL); LEUFKENS, Timmy Robertus Maria, 5656 AE Eindhoven (NL); VAN EE, Raymond, 5656 AE Eindhoven (NL); LAMERICHS, Mathias Johannes Nicolaas, 5656 AE Eindhoven (NL); MATTERS, Marco, 5656 AE Eindhoven (NL); MENA BENITO, Maria Estrella, 5656 AE Eindhoven (NL); HUIJBERS, Willem, 5656 AE Eindhoven (NL); WESTERINK, Joanne Henriëtte Desirée Monique, 5656 AE Eindhoven (NL); DENISSEN, Adrianus Johannes Maria, 5656 AE Eindhoven (NL)
(74) Representative: Philips Intellectual Property & Standards

(57) **Abstract**

The present invention relates to attention bias modification treatment. In order to improve the treatment effect, an ABM system is proposed with a neural brain response monitoring device, such as functional magnetic resonance imaging (fMRI), magnetoencephalography (MEG), and/or electroencephalography (EEG), to provide real-time information regarding neural brain responses in order to personalize and optimize the ABM treatment.

## Description

### FIELD OF THE INVENTION

The present invention relates to attention bias modification treatment, and in particular to an attention bias modification treatment system, an attention bias modification treatment method, a computer program element, and a computer readable medium.

### BACKGROUND OF THE INVENTION

Individuals suffering from a mental disorder (e.g. a major depressive disorder, an anxiety disorder, or insomnia, or substance abuse disorder) are currently being treated with i) psychopharmaca (e.g. SSRI's, benzodiazepines) and/or psychotherapy (e.g. CBT). While both treatment methods have proven therapeutic effects, both methods also have their limitations and even negative effects. For instance, many medicinal drugs are known to also have unwanted side-effects and psychotherapy is costly and patients often show decreased treatment compliance.

As a response, attention bias modification (ABM) therapy has been shown to yield similar efficacy with the advantage that it is cost-effective and easy to use for patients. After all, many patients with aforementioned mental disorders display a certain attentional bias towards stimuli related to the underlying complaints of their disorder (e.g. bias towards negatively annotated stimuli -images, faces, words, phrases - in individuals having a depression, bias towards stimuli annotated with their preoccupation such as simple words like bedroom, pillow, or night in the case of insomnia, or bias towards market square or crowded pubs in anxiety).

However, a downfall of ABM therapy, as is the case with psychopharmaca and psychotherapy, is that still the treatment effect is at most 60%.

### SUMMARY OF THE INVENTION

There may be a need to improve the ABM treatment effect.

The object of the present invention is solved by the subject-matter of the independent claims, wherein further embodiments are incorporated in the dependent claims. It should be noted that the following described aspects of the invention apply also for the attention bias modification treatment system, the attention bias modification treatment method, the computer program element, and the computer readable medium.

According to a first aspect of the present invention, there is provided an ABM system. The ABM system comprises a treatment system and a neural brain response monitoring device. The treatment system comprises a controller configured to send a control signal indicative of an ABM task. The treatment system also comprises a stimulation device configured to apply at least one sensory stimulus over a human subject in response to the control signal, said at least one sensory stimulus being associated with at least one attentional bias, wherein the at least one attentional bias represents impaired attentional engagement with or disengagement from a sensory stimulus. The treatment system further comprises a measuring device configured to measure at least one behavioural response of the human subject to the at least one applied sensory stimulus. The neural brain response monitoring device is configured to monitor a brain activation metric of the human subject to the at least one applied sensory stimulus. The ABM task is adaptable to the monitored brain activation metric. Alternatively or additionally, a neurofeedback device is provided for generating a feedback signal indicative of the monitored brain activation metric.

In other words, an ABM system is proposed with a neural brain response monitoring device, such as functional magnetic resonance imaging (fMRI), magnetoencephalography (MEG), and electroencephalography (EEG), to provide real-time information regarding neural brain responses in order to personalize and optimize the ABM treatment.

Based on the neural brain response to a presented sensory stimulus that is measured by scanning the functional activation of a relevant structure (e.g. amygdala), the relative distribution of e.g. negatively, positively, and neutrally annotated stimuli may be adapted more precisely and more personally, instead of the current automated, fixed way of ramping up the positively annotated stimuli.

Alternatively or additionally, the neurofeedback device may provide feedback to a patient and the patient may be instructed to attempt to lower or increase the neuronal response to that stimulus to optimize the ABM treatment.

According to an embodiment of the present invention, the controller is configured to modify the ABM task according to the monitored brain activation metric.

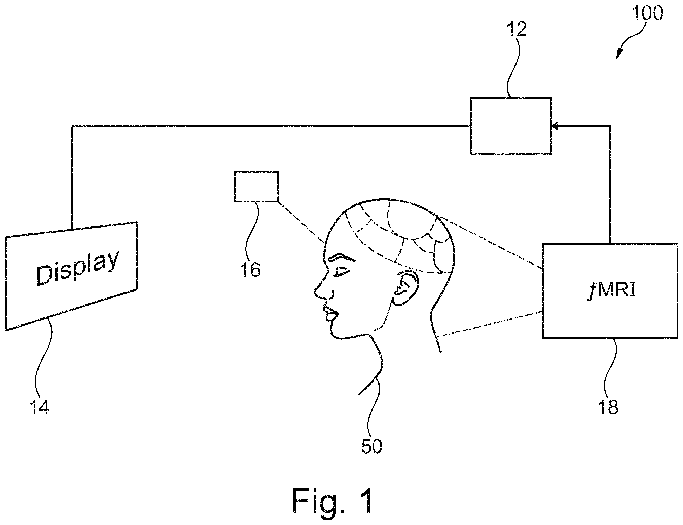

For example, the ABM task may be done automatically by an optimization algorithm implemented in the controller. This will be explained hereafter and in particular with respect to the example illustrated in Fig. 1.

According to an embodiment of the present invention, the controller is configured to modify one or more of the following parameters to adapt the ABM task:
- intensity of the at least one applied sensory stimulus;
- valence of the at least one applied sensor stimulus; and/or
- content and/or semantics of the at least one applied sensor stimulus.

According to an embodiment of the present invention, the controller is configured to add a neurofeedback task to the ABM task. In the neurofeedback task the human subject is instructed to attempt to lower or increase the brain activation metric of the human subject to the at least one applied sensory stimulus.

In other words, the ABM therapy may be combined with an actual real-time neurofeedback task based on the neural response of the sensory stimuli that are presented during the ADM task. This will be explained hereafter and in particular with respect to the example illustrated in Fig. 1.

According to an embodiment of the present invention, the neural brain response monitoring device is configured to monitor one or more of: (i) one or more specific brain areas, (ii) brain activation networks, and (iii) connectivity between brain areas that are related to specific forms of affective behavioural states to determine the brain activation metric of the human subject to the at least one applied sensory stimulus.

According to an embodiment of the present invention, the feedback signal is presented by a second modality that is different from a first modality for presenting the at least one sensory stimulus.

For example, the ABM task may be a visuospatial task where two visual stimuli are presented simultaneously at two separate spatial locations, and the two visual stimuli can be e.g. facial expressions with two different emotional valences, one with a negative valence, and one with a neutral valence. Then the neural brain response may be a feedback to the human subject or a user of the system, in a modality that is not visuospatial. An example of a non-visuospatial modality may be e.g. the pitch of a sound where an increasing pitch specifies intensification of the brain activity metric, and a decreasing pitch is indicative of a lowering of the brain response.

In this way, the feedback signal will not distract the attention of the human subject away from the attention task.

According to an embodiment of the present invention, the controller is configured such that any changes in the neurofeedback response to the human subject are timed between ABM tasks, such that the human subject is less distracted from the ABM tasks.

In other words, the provision of neurofeedback to the user of the system, or the adaptation of the ABM task according to the measured brain activation metric, may be timed, such that it does not distract the human subject.

According to an embodiment of the present invention, the neural brain response monitoring device comprises a neuroimaging device. The neuroimaging device may comprise one or more of: fMRI, MEG, EEG, functional near-infrared spectroscopy (fNIR), optically-pumped magnetometers (OPM), and functional ultrasound (fUS).

According to an embodiment of the present invention, the controller is configured to determine, based on the monitored brain activation metric, whether to continue the ABM task or abort the ABM task. Alternatively or additionally, the controller is configured to determine, based on the monitored brain activation metric, whether to adapt the ABM task.

In other words, the controller may determine not only to continue or abort, but also how to adapt the ABM task, in case we apply the adaptation of the ABM task, for example by selecting the next sensory stimuli that will be applied in the ABM task, or in an advanced version by generating (e.g. in real time) the next sensory stimuli that will be applied in the ABM task.

In case of mere provision of neurofeedback to the human subject, without adapting the ABM task, the task of the controller may be to decide when and how to provide the measured neurofeedback (e.g. in real time during the ABM task, or after an ABM task block, etc.) and in which modality (e.g. visual or auditory feedback).

According to an embodiment of the present invention, the treatment system further comprises a behavioural response feedback device configured to provide an attentional bias feedback based on the at least one measured behavioural response.

According to an embodiment of the present invention, the at least one measured behavioural response of the human subject comprises at least one of:
- reaction time response to the ABM task;
- gaze time, gaze position, and/or gaze location; and
- a number of fault responses to the ABM task.

According to a second aspect of the present invention, there is provided an attention bias modification treatment (ABM) method that comprises:
a) sending, by a controller, a control signal indicative of an ABM task;
b) applying, by a stimulation device, at least one sensory stimulus over a human subject in response to the control signal, said at least one sensory stimulus being associated with at least one attentional bias, wherein the at least one attentional bias represents impaired attentional engagement with or disengagement from a sensory stimulus;
c) measuring, by a measuring device, at least one behavioural response of the human subject to the at least one applied sensory stimulus;
d) monitoring, by a neural brain response monitoring device, an brain activation metric of the human subject to the at least one applied sensory stimulus; and
e) adapting the ABM task to the monitored brain activation metric; and/or
   - generating, by a neurofeedback device, a feedback signal indicative of the monitored brain activation metric.

According to an embodiment of the present invention, the feedback signal is presented by a second modality that is different from a first modality for presenting the at least one sensory stimulus.

According to a third aspect of the present invention, there is provided a computer program element for controlling an attention bias modification treatment, ABM, system according to the second aspect and any associated example, which when being executed by a processor is configured to carry out the method according to the third aspect and any associated example.

According to a fourth aspect of the present invention, there is provided a computer readable medium having stored thereon the program element.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 schematically shows an example of an ABM system.
Fig. 2 schematically shows a further example of an ABM system.
Fig. 3 is a flowchart of a method, in accordance with an embodiment.

### DETAILED DESCRIPTION OF EMBODIMENTS

Individuals having a depression may display a certain attentional bias towards negatively, positively, and neutral annotated stimuli. The attentional bias could also be related to the patient's worry, preoccupation, or complaints. Those words do not necessarily have a positive or negative value. They have a meaning for the patient that is related to their underlying issue and therefore create an attentional bias.

Fig. 1 illustrates an example of an ABM system 100 for treating individuals suffering from a mental disorder (e.g. a major depressive disorder, an anxiety disorder, or insomnia, or substance abuse disorder). The ABM system comprises a controller 12, a stimulation device 14, a measuring device 16, and a neural brain response monitoring device 18.

The controller 12 is configured to send a control signal indicative of an ABM task.

In an example, the ABM task may be a visual-probe task to reduce attentional bias (AB) to threat. On each trial, two cues appear simultaneously in different regions of a computer screen, typically for 500 milliseconds (ms). On critical trials, one cue is threat-related (e.g., angry face) and the other non-threat (e.g., neutral face). A target-probe (e.g., dot or letter) then replaces one of the cues, either in the location just occupied by the threat cue (threat-congruent trial) or opposite location (threat-incongruent trial). The task of the participant is to respond as quickly as possible after detection of the probe. In the attention bias modification trials, the probe will always (in 100% of trials) be shown at the spatial location of the neutral stimulus such that the participant learns to pay most attention to the neutral stimulus, in order to be faster in detecting the probe.

In another example, the ABM task may be a modified Stroop task. This task requires subjects to name the colour of a word while ignoring the meaning of that word. A consistent finding in Stroop studies with anxious patients is that their colour naming of threatening words is slower than that of neutral words.

In a further example, the ABM task may be a dichotic audio task with one neutral voice and one angry voice in left or right ear.

The controller 12 may be implemented in numerous ways (e.g., such as with dedicated hardware) to perform various functions discussed herein. A "processor" is one example of a controller, which employs one or more microprocessors that may be programmed using software (e.g., microcode) to perform various functions discussed herein. The controller may be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).

The stimulation device 14 is configured to apply at least one sensory stimulus over a human subject 50 in response to the control signal. The at least one sensory stimulus is associated with at least one attentional bias. The at least one attentional bias represents impaired attentional engagement with or disengagement from a sensory stimulus.

For example, the human subject 50 may be given one or more ABM tasks, such as a set of ABM tasks, to perform. In each ABM task, the stimulation device 14 may be configured to apply one or more sensory stimuli on or presented to the human subject 50.

In the example illustrated in Fig. 1, the stimulation device 14 is a visual stimulation device. The visual stimulation device may be a screen, such as a computer monitor or a television, for displaying any combination of still images and video for predetermined durations on predetermined areas of the screen. When using visual tasks, in order to assess the ABM the patient attention to the visual probe is represented by the response time of the patient to the appearance of the probe. This is currently done outside the scanner, in a controlled laboratory like situation. Therefore, in the example of Fig. 1, it may be beneficial to transfer such a laboratory-based task into the equivalent scanner based task. This may be achieved by ensuring that both the visual stimuli and patient response are made compatible with the scanner and the scan protocol. For example, the visual stimuli may be brought into the scanner using a mirror arrangement to allow the patient to view an (inverted) display in the mirror, by a projection of the visual stimuli onto the bore of the MRI, by providing the patient with MRI compatible head mounted displays etc.

In another example (not shown), the stimulation device 14 may be an auditory stimulation device, such as a speaker connected to an audio source and outputting predetermined sounds at predetermined timings according to the control signal.

In a further example (not shown), the stimulation device 14 may be a tactile stimulation device, such as a vibrating device that is touching (or very close to) the human subject and in response to the control signal, vibrates at predetermined vibration strength and frequency and at predetermined timings.

The measuring device 16 is configured to measure at least one behavioural response of the human subject to the at least one applied sensory stimulus.

In the example of Fig. 1, the measuring device 16 is an eye-tracking system. The eye-tracking system may use laser technology to track the pupils of the human subject and therefore deduce the gaze time, gaze position, gaze location, and/or other such parameters. For example, the eye-tracking system may be configured to measure where individuals direct their gaze (i.e. gaze position) when they are presented with a visual stimulus on a computer screen, providing a direct assessment of attention allocation.

In another example (not shown), the measuring device 16 may be configured to measure a reaction time response to the ABM task. The reaction time is the amount of time it takes for the human subject to respond to a stimulus that is presented to him/her. These are usually measured in milliseconds.

In a further example (not shown), the measuring device 16 may be configured to measure a number of fault responses to the ABM task.

Based on the at least one measured behavioural response, an attentional bias feedback may be provided by a behavioural response feedback device (not shown), such as a display or a light emitting device.

The neural brain response monitoring device 18 is configured to monitor a brain activation metric of the human subject to the at least one applied sensory stimulus. In other words, the neural brain response monitoring device 18 provides real-time information regarding neural brain responses to the at least one applied sensory stimulus.

The neural brain response monitoring device 18 may be used to monitor the brain activation metric in one or more specific brain areas.

Alternatively or additionally, the neural brain response device 18 may be configured to for example monitor brain activation networks and connectivity between brain areas.

The brain activation metric is a metric that quantifies the measured neuronal activity in response to a stimulus.

In the example illustrated in Fig. 1, the neural brain response device 18 is a functional Magnetic Resonance Imaging (fMRI) device, which is a non-invasive tool used to measure neuronal activity in response to a stimulus, or task. The fMRI device does not directly measure the activity of neurons, i.e. action potentials of single cell neurons, but relies on the coupling between neuronal metabolism and blood flow. Functional MRI sequences typically employ the blood oxygen level-dependent (BOLD) contrast to estimate brain activity.

In another example (not shown), the neural brain response device 18 may be a device for performing other methods of non-invasive neuromonitoring. For example, electroencephalography (EEG), magnetoencephalography (MEG), optically-pumped magnetometers (OPM), or motor-evoked responses may be used to estimate the amount of neuronal activity induced by the ABM task. These neuromonitoring methods are not based on brain hemodynamics, but measure the brain's electrical currents.

The real-time information regarding neural brain responses may be used to personalize and optimize the ABM treatment using at least one of the following approaches.

### ABM task adaptable to the monitored brain activation metric

The monitored brain activation metric may be used to modify the ABM treatment.

In an example, the immediate neuronal activity, such as immediate emotional response, to a presented sensory stimulus may be measured by scanning the functional activation of a relevant structure (e.g. amygdala). Based on the activation, the sensory stimuli in the ABM task may be adapted in terms of intensity, i.e. higher valence of the stimuli in either positive (neutral) or negative direction depending on the level of functional activation of the relevant structure. The response of the human subject could be probed by changing the valence of the stimulus. For example, an absent or minimal functional response may be the result of a too neutral stimulus. By presenting a more negative stimulus, a functional response may be detected showing that the treatment needs to be continued.

In another example, the immediate neuronal activity, such as immediate emotional response, to a presented sensory stimulus may be measured by scanning the functional activation of a relevant structure (e.g. amygdala). Based on the activation, the stimuli in the ABM task are adapted in terms of content and semantics. Based on the activation the stimuli in the ABM task are adapted in terms of content, e.g. more negatively or positively annotated stimuli depending on the level of functional activation of the relevant structure. The response of the human subject could be probed by changing the content or semantics of the stimulus. For example, an absent or minimal functional response may be the result of a too neutral stimulus. By presenting a more negatively annotated stimulus, a functional response may be detected showing that the treatment needs to be continued.

In a further example, a neurofeedback task may be added to the ABM task. In the neurofeedback task the human subject may be instructed to attempt to lower or increase the brain activation metric of the human subject to the at least one applied sensory stimulus. In other words, the ABM therapy may be combined with an actual real-time neurofeedback task based on the neural response of the sensory stimuli that are presented during the ADM task. For example, if the human subject shows an extreme response to a specific negatively annotated stimulus, this stimulus may be used in the neurofeedback task that is presented directly following the ABM task. In the neurofeedback task, the human subject may be instructed to attempt to lower the neuronal response to that stimulus. If a certain decreased level of neuronal response is reached, the next ABM session may be started containing that stimulus.

In a further example, the direct effect of the ABM treatment may be monitored. A decision based on the neuronal response may be made whether to continue the ABM task or to abort the ABM task. For example, the ABM task may be continued if a treatment effect is detected and prolongation of the therapy would result in improvement of symptoms. For example, the ABM task may be aborted, if the optimal treatment effect is reached or it is not useful to continue the therapy as no treatment effect is detected.

Based on the neural brain response to a presented sensory stimulus that is measured by scanning the functional activation of a relevant structure (e.g. amygdala), the relative distribution of negatively, positively, and neutrally annotated stimuli may be adapted more precisely and more personally, instead of the current automated, fixed way of ramping up the positively annotated stimuli.

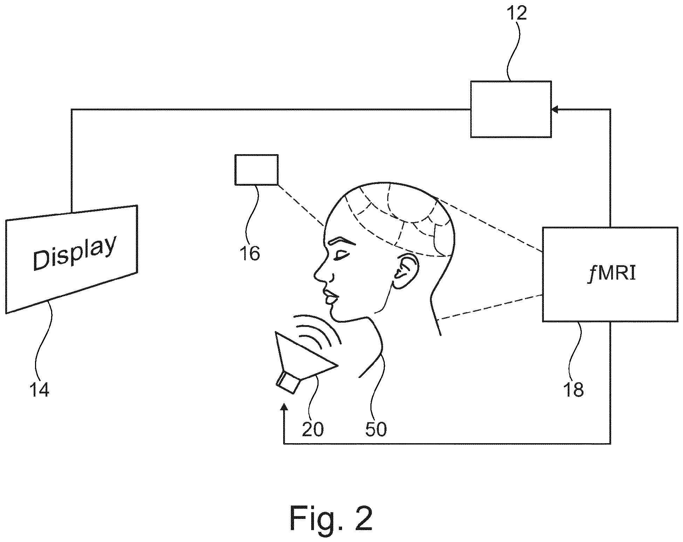

The above-described adaptions of the ABM task may be done automatically by an optimization algorithm implemented in the controller 12, which analyzes the data collected by the neural brain response device 18. Alternatively, a therapist who observes the brain response activates during the treatment may modify the ABM task manually. This may be done with the help of a feedback signal indicative of the monitored brain activation metric in the example of Fig. 2

### Feedback signal indicative of the monitored brain activation metric

Fig. 2 illustrates another example of the ABM system. In the example of Fig. 2, a neurofeedback device 20 is provided for generating a feedback signal indicative of the monitored brain activation metric.

In some examples, the neurofeedback device 20 may provide feedback to a therapist. In this case, the controller 12 may comprise a user interface (not shown) to allow the therapist to adapt or re-adapt some features of the ABM task. The term "user interface" as used herein refers to an interface between a human user or operator and one or more devices that enables communication between the user and the device(s). Examples of user interfaces that may be employed in various implementations of the present disclosure include, but are not limited to, switches, potentiometers, buttons, dials, sliders, track balls, display screens, various types of graphical user interfaces (GUIs), touch screens, microphones and other types of sensors that may receive some form of human-generated stimulus and generate a signal in response thereto.

Alternatively or additionally, the neurofeedback device 20 may provide feedback to the human subject 50 and the human subject 50 may be instructed to attempt to lower or increase the neuronal response to that stimulus to optimize the ABM treatment.

In an example, the neurofeedback device 20 may be a thermometer with increasing/decreasing bar chart to represent the monitored neuronal activity.

In an example, the neurofeedback device 20 may be an auditory feedback device for generating an audio signal. The frequency, intensity, and/or repeat frequency of the audio signal may represent the monitored neuronal activity.

In an example, the neurofeedback device 20 may be a tactile feedback device, e.g. a vibrating device, for generating a tactile signal. The frequency, intensity, spatial extent, and/or pattern of the tactile signal may represent the monitored neuronal activity.

In an example, the neurofeedback device 20 may be a heat feedback device, such as a heating element. The power, spatial extent, and/or pattern of the heating element may represent the monitored neuronal activity.

In an example, the neurofeedback device 20 may be a visual feedback device, such as a display. The colour, texture, and/or brightness of the display may represent the monitored neuronal activity.

Preferably, the feedback signal may be presented by a second modality that is different from a first modality for presenting the at least one sensory stimulus.

In the example of Fig. 1, the stimulation device 14 is a display for generating visual stimuli. It is required to provide the neurofeedback to the human subject 50 in real time as described above. However, in the example of Fig. 1, some feedback methods, such as the thermometer approach, may be unsuitable. Specifically, as it is required that the attention of the human subject 50 is on the visual stimuli followed by the probe, any features on the display image (e.g. the thermometer), which distract visual attention from the desired stimuli, may inevitably cause uncontrolled delays in the response of the human subject 50 to the probe. In particular, if the human subject 50 is paying attention to the thermometer in order to see if their neuronal activity is under control, the human subject 50 will not be giving his/her full attention to the visual task. Consequently, the recorded response time may not reflect the true response time in the absence of the visual representation of the brain activity (i.e. the thermometer).

For this reason, when using a visual response time task, it is proposed that a neural feedback approach is used which provides the required neural feedback without affecting the response time. Thus, the feedback signal may be presented by a second modality different from the first modality used for presenting the visual response task. Exemplary second modalities may include, but are not limited to, auditory feedback, tactile feedback, heat feedback, and non-spatial visual feedback. In this way, the human subject will be giving his/her full attention to the visual task and the recorded response time will reflect the true response time in the absence of the visual representation of the brain activity (i.e. the thermometer).

It is also noted that the time constant of the neurofeedback response is usually considerably longer than the response time of the ABM response, whereby slower feedback modalities - such as the heat feedback - are applicable.

Optionally, the controller 12 may be configured such that any changes in the neurofeedback response to the human subject are timed between ABM tasks, such that the human subject is less distracted from the ABM tasks. This may be done by having different time blocks in the ABM tasks. In an example, the ABM task and the induced neurofeedback response may be in the same time block. In this way, the neurofeedback response is provided in real-time. In another example, the induced neurofeedback response may be provided in the next time block. In this way, the neurofeedback response is provided with a delay.

The aforementioned ABM system 100 may be used to train the subject -or calibrate the system- in one or more sessions, in such a way that the ABM method can be used without the neural brain response monitoring device, or with another (neural brain response) monitoring device that was not used during the training or calibration. This second (calibrated) device can be a cheaper sensor, e.g. wearable sensor, that can be used for ABM neurofeedback after training. For example, a subject is using the ABM system in the fMRI. Simultaneously, another monitoring device (e.g. EEG, skin conductance or eye-tracking pupil diameter) may be used on the subject. After one or more session the ABM system, the subject and the system are "calibrated" for optimal effectiveness. Further sessions of ABM may be done outside the fMRI using no or the second monitoring device, using the same settings/stimuli as in the calibration session.

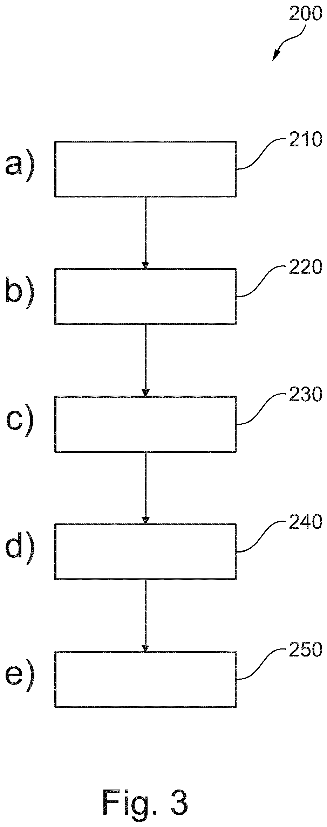

Fig. 3 shows a flowchart of an ABM treatment method 200.

In a first step 210, i.e. step a), a controller sends a control signal indicative of an ABM task. Exemplary ABM tasks may include, but are not limited to, modified Stroop tasks, visual-probe tasks, and dichotic audio tasks.

In a second step 220, i.e. step b), a stimulation device applies at least one sensory stimulus over a human subject in response to the control signal. The at least one sensory stimulus being associated with at least one attentional bias. The at least one attentional bias represents impaired attentional engagement with or disengagement from a sensory stimulus. In each task, one or more stimuli may be applied on or presented to the subject such as visual, auditory, olfactory and/or tactile stimulus, through one or more stimulation devices such as a screen, a speaker, a light emitting source, vibrating devices for tactile stimulation, and the like.

In a third step 230, i.e. step c), a measuring device measures at least one behavioural response of the human subject to the at least one applied sensory stimulus. Exemplary behavioural responses of the human subject may include, but are not limited to, reaction time response to the ABM task; gaze time, gaze position, and/or gaze location; and a number of fault responses to the ABM task.

In a fourth step 240, i.e. step d), a neural brain response monitoring device monitors a brain activation metric of the human subject to the at least one applied sensory stimulus. The brain activation metric indicates the neuronal activity.

The neural brain response monitoring device may be configured to monitor one or more of: (i) one or more specific brain areas, (ii) brain activation networks, and (iii) connectivity between brain areas that are related to specific forms of affective behavioural states to determine the brain activation metric of the human subject to the at least one applied sensory stimulus.

The neural brain response monitoring device may comprise one or more of: fMRI, MEG, EEG, fNIR, OPM, and fUS.

In a fourth step 250, i.e. step e), the ABM task is adapted to the monitored brain activation metric with at least one of the following approaches.

In an example, the controller is configured to adapt the ABM task by modifying the intensity of the at least one applied sensory stimulus.

In an example, the controller is configured to adapt the ABM task by modifying the valence of the at least one applied sensor stimulus.

In a further example, the controller is configured to adapt the ABM task by modifying the content and/or semantics of the at least one applied sensor stimulus.

Alternatively or additionally, a neurofeedback device may be provided for generating a feedback signal indicative of the monitored brain activation metric. Exemplary feedback signals may include, but are not limited to, visual signals, auditory signals, tactile signals, etc.

Preferably, the feedback signal is presented by a second modality that is different from a first modality for presenting the at least one sensory stimulus. For example, if the sensory stimulus is a visual signal, the feedback signal may be e.g. an auditory signal, a tactile signal, or other signals that have a modality different from the visual signal. In this way, the human subject is less distracted from the at least one sensory stimulus of the ABM task.

It should also be understood that, unless clearly indicated to the contrary, in any methods claimed herein that include more than one step or act, the order of the steps or acts of the method is not necessarily limited to the order in which the steps or acts of the method are recited.

The above-described system and method may be applied to psychiatric treatment in the radiology department, e.g. of Major Depressive Disorder (MDD), Anxiety Disorder, Obsessive Compulsive Disorder, Substance Abuse Disorder, etc.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one. "

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. An attention bias modification treatment, ABM, system (100), comprising:
- a controller (12) configured to send a control signal indicative of an ABM task;
- a stimulation device (14) configured to apply at least one sensory stimulus over a human subject in response to the control signal, said at least one sensory stimulus being associated with at least one attentional bias, wherein the at least one attentional bias represents impaired attentional engagement with or disengagement from a sensory stimulus;
- a measuring device (16) configured to measure at least one behavioural response of the human subject to the at least one applied sensory stimulus; and
- a neural brain response monitoring device (18) configured to monitor a brain activation metric of the human subject to the at least one applied sensory stimulus,
wherein the ABM task is adaptable to the monitored brain activation metric and/or a neurofeedback device (20) is provided for generating a feedback signal indicative of the monitored brain activation metric.

2. The ABM system according to claim 1,
wherein the controller is configured to modify the ABM task according to the monitored brain activation metric.

3. The ABM system according to claim 2,
wherein the controller is configured to modify one or more of the following parameters to adapt the ABM task:
- intensity of the at least one applied sensory stimulus;
- valence of the at least one applied sensor stimulus; and/or
- content and/or semantics of the at least one applied sensor stimulus.

4. The ABM system according to claim 2 or 3,
wherein the controller is configured to add a neurofeedback task to the ABM task, wherein in the neurofeedback task the human subject is instructed to attempt to lower or increase the brain activation metric of the human subject to the at least one applied sensory stimulus.

5. The ABM system according to any one of the preceding claims,
wherein the neural brain response monitoring device is configured to monitor one or more of: (i) one or more specific brain areas, (ii) brain activation networks, and (iii) connectivity between brain areas that are related to specific forms of affective behavioural states to determine the brain activation metric of the human subject to the at least one applied sensory stimulus.

6. The ABM system according to any one of the preceding claims,
wherein the feedback signal is presented by a second modality that is different from a first modality for presenting the at least one sensory stimulus.

7. The ABM system according to any one of the preceding claims,
wherein the controller is configured such that any changes in the neurofeedback response to the human subject are timed between ABM tasks, such that the human subject is less distracted from the ABM tasks.

8. The ABM system according to any one of the preceding claims,
wherein the neural brain response monitoring device comprises a neuroimaging device.

9. The ABM system according to any one of the preceding claims,
wherein the controller is configured to determine one or more of the following based on the monitored brain activation metric:
- whether to continue the ABM task or abort the ABM task; and
- whether to adapt the ABM task.

10. The ABM system according to any one of the preceding claims, further comprising:
a behavioural response feedback device configured to provide an attentional bias feedback based on the at least one measured behavioural response.

11. The ABM system according to any one of the preceding claims,
wherein the at least one measured behavioural response of the human subject comprises one or more of:
- reaction time response to the ABM task;
- gaze time, gaze position, and/or gaze location; and
- a number of fault responses to the ABM task.

12. An attention bias modification, ABM, treatment method (200), comprising:
a) sending (210), by a controller, a control signal indicative of an ABM task;
b) applying (220), by a stimulation device, at least one sensory stimulus over a human subject in response to the control signal, said at least one sensory stimulus being associated with at least one attentional bias, wherein the at least one attentional bias represents impaired attentional engagement with or disengagement from a sensory stimulus;
c) measuring (230), by a measuring device, at least one behavioural response of the human subject to the at least one applied sensory stimulus;
d) monitoring (240), by a neural brain response monitoring device, an brain activation metric of the human subject to the at least one applied sensory stimulus; and
e) adapting (250) the ABM task to the monitored brain activation metric; and/or
- generating, by a neurofeedback device, a feedback signal indicative of the monitored brain activation metric.

13. Method according to claim 12, wherein the feedback signal is presented by a second modality that is different from a first modality for presenting the at least one sensory stimulus.

14. A computer program element for controlling an attention bias modification treatment, ABM, system according to any one of claims 1 to 11, which when being executed by a processor is configured to carry out the method according to claim 12 or 13.

15. A computer readable medium having stored thereon the program element of claim 14.
